# EUROPEAN PATENT SPECIFICATION

(11) **EP 4 470 115 B1**
(45) Date of publication and mention of the grant of the patent: **15.01.2025**
(21) Application number: 23793624.0
(22) Date of filing: 22.09.2023
(51) Int. Cl.: H04B 5/79, H02J 7/00, H02J 50/80, H02J 50/12, G04G 21/04, G04G 19/00, G04C 10/00, A61B 5/00

(54) **SELECTIVE DATA TRANSFER FOR EFFICIENT WIRELESS CHARGING**
SELEKTIVE DATENÜBERTRAGUNG FÜR EFFIZIENTES DRAHTLOSES LADEN
TRANSFERT SÉLECTIF DE DONNÉES POUR UNE RECHARGE SANS FIL EFFICACE

(30) Priority: 23.09.2022 US 202263409356 P
(43) Date of publication of application: 04.12.2024
(73) Proprietor: Whoop, Inc., Boston, MA 02215 (US)
(72) Inventor: SORENTI, Elizabeth, Anne, Boston, MA 02215 (US); MARTINS, Brian, Anthony, Boston, MA 02215 (US); GANTI, Aasrith, San Jose, CA 95134 (US); WALSH, Jacob, Sheridan, Boston, MA 02215 (US)
(74) Representative: Black, Diego
(86) International application number: PCT/US2023/074835
(87) International publication number: WO 2024/064859

(56) References cited:
- WO-A1-2022/082077
- US-A1- 2013 113 422
- US-A1- 2015 054 455
- US-A1- 2016 126 639
- US-A1- 2021 210 992

## Description

### CROSS-REFERENCE TO RELATED APPLICATIONS

This application claims priority to U.S. Provisional Pat. App. No. 63/409,356 filed on September 23, 2022.

### TECHNICAL FIELD

The present disclosure generally relates to selective wireless transmission of data and power in a near field system.

### BACKGROUND

A device, such as a physiological monitor, may include circuitry for both wireless charging and for wireless data transmission. The device may, for example, use near field wireless power and data transfer systems conforming to one or more Near Field Communication (NFC) design specifications and protocols. However, in such a device, a data transfer process can interfere with a wireless power transfer process, thereby decreasing efficiency when charging the device. There remains a need for improved charging efficiency for devices that wirelessly transfer both data and power.

Patent application US 2013/113422 A1 relates to a wireless power receiver and a control method thereof.

Patent application US 2015/054455 A1 relates to a receiver for a wireless charging system.

Patent application US 2021/210992 A1 relates to systems and methods for wireless power and data transfer that utilize multiple antennas on the receiver side.

Patent application US 2016/126639 A1 relates to a coil structure and a wireless power receiving apparatus including the same.

International publication WO 2022/082077 A1 relates to physiological monitoring systems and arrangements for deploying and using same.

### SUMMARY

A device includes a power antenna and a data antenna, along with a switch operable to selectively connect the data antenna to data circuitry under various conditions. 2. The switch is to decouple the data antenna when charging circuitry is receiving power through the power antenna, or under other conditions indicating that charging may be available or desired, thereby allowing for relatively unimpeded and efficient wireless charging of a device. The switch is closed to enable use of the data antenna by data circuitry when wireless charging activity is not present, or when other conditions indicate that wireless data is available for the device.

In an aspect, a system disclosed herein includes:
a power antenna; a wireless power receiver coupled to the power antenna; a data antenna; a wireless data receiver coupled to the data antenna; a switch selectively coupling the data antenna to the wireless data receiver; and
control circuitry configured to operate the switch to disconnect the data antenna from the wireless data receiver in response to power being received by the wireless power receiver through the power antenna.

Implementations may include one or more of the following features. The control circuitry may be configured to operate the switch to connect the data antenna to the wireless data receiver when an amount of power received by the wireless power receiver is below a predetermined threshold. The control circuitry may be configured to default to a power receive mode with the switch in an open position. The control circuitry may be configured to respond to a data availability event by: connecting the data antenna to the wireless data receiver using the switch, and actively pinging for a readable data tag with the wireless data receiver. According to the claimed invention, the data availability event includes at least one of a detection of a contact of at least part of the system with skin of a user or a detection of a garment pocket around at least part of the system. The system may include a physiological monitor. The physiological monitor may include a battery charged at least partially by the wireless power receiver. The physiological monitor may include a controller configured to operate the physiological monitor in response to data received by the wireless data receiver. The data may include at least one of a garment type and a body location. The power antenna may be a planar coil antenna. The data antenna may be a planar coil antenna. The data antenna may be in a parallel plane to the power antenna. The data antenna may overlap the power antenna along an axis normal to a plane of the data antenna. The data antenna may be concentric with the power antenna. The wireless power receiver may include a Near Field Communication power receiver and the wireless data receiver includes a Near Field Communication data tag reader.

In an aspect according to the claimed invention, a method disclosed herein includes detecting a data availability event on a device, where: the data availability event indicates an availability of data wirelessly available for the device; the device includes a wireless power receiver coupled to a power antenna; the device includes a wireless data receiver coupled to a data antenna; and the device includes a switch selectively coupling the wireless data receiver to the data antenna. The method also includes, in response to detecting the data availability event, operating the switch to selectively couple the wireless data receiver to the data antenna, and initiating a connection between the device and a wireless data source through the data antenna. Furthermore, according to the claimed invention, the data availability event includes detection of insertion of the device into a pocket of a garment adjacent to a data tag. Other embodiments of this aspect include corresponding computer systems, apparatus, and computer programs recorded on one or more computer storage devices, each configured to perform the actions of the methods.

Implementations may include one or more of the following features. The data availability event may include a detection of an amount of power received by the wireless power receiver below a predetermined threshold. The data availability event may include a detection of contact with a user. Implementations of the described techniques may include hardware, a method or process, or computer software on a computer-accessible medium.

### BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.
Fig. 1 shows a physiological monitoring device.
Fig. 2 illustrates a physiological monitoring system.
Fig. 3 shows a smart garment system.
Fig. 4 illustrates circuitry for wireless power and data transmission.
Fig. 5 shows a printed circuit board layout.
Fig. 6 is a flow chart of a method for wireless power and data transmission.
Fig. 7 illustrates circuitry for wireless power and data transmission.

### DESCRIPTION

The embodiments will now be described more fully hereinafter with reference to the accompanying figures, in which preferred embodiments are shown. The foregoing may, however, be embodied in many different forms and should not be construed as limited to the illustrated embodiments set forth herein. Rather, these illustrated embodiments are provided so that this disclosure will convey the scope to those skilled in the art.

References to items in the singular should be understood to include items in the plural, and vice versa, unless explicitly stated otherwise or clear from the text. Grammatical conjunctions are intended to express any and all disjunctive and conjunctive combinations of conjoined clauses, sentences, words, and the like, unless otherwise stated or clear from the context. Thus, the term "or" should generally be understood to mean "and/or" and so forth.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the embodiments. No language in the specification should be construed as indicating any unclaimed element as essential to the practice of the embodiments.

In the following description, it is understood that terms such as "first," "second," "top," "bottom," "up," "down," "above," "below," and the like, are words of convenience and are not to be construed as limiting terms unless specifically stated to the contrary.

The term "user" as used herein, refers to any type of animal, human or non-human, whose physiological information may be monitored using an exemplary wearable physiological monitoring system.

The term "continuous," as used herein in connection with heart rate data, refers to the acquisition of heart rate data at a sufficient frequency to enable detection of individual heartbeats, and also refers to the collection of heart rate data over extended periods such as an hour, a day or more (including acquisition throughout the day and night). More generally with respect to physiological signals that might be monitored by a wearable device, "continuous" or "continuously" will be understood to mean continuously at a rate and duration suitable for the intended time-based processing, and physically at an inter-periodic rate (e.g., multiple times per heartbeat, respiration, and so forth) sufficient for resolving the desired physiological characteristics such as heart rate, heart rate variability, heart rate peak detection, pulse shape, and so forth. At the same time, continuous monitoring is not intended to exclude ordinary data acquisition interruptions such as temporary displacement of monitoring hardware due to sudden movements, changes in external lighting, loss of electrical power, physical manipulation and/or adjustment by a wearer, physical displacement of monitoring hardware due to external forces, and so forth. It will also be noted that heart rate data or a monitored heart rate, in this context, may more generally refer to raw sensor data such as optical intensity signals, or processed data therefrom such as heart rate data, signal peak data, heart rate variability data, or any other physiological or digital signal suitable for recovering heart rate information as contemplated herein. Furthermore, such heart rate data may generally be captured over some historical period that can be subsequently correlated to various other data or metrics related to, e.g., sleep states, recognized exercise activities, resting heart rate, maximum heart rate, and so forth.

The term "computer-readable medium," as used herein, refers to a non-transitory storage media such as storage hardware, storage devices, computer memory that may be accessed by a controller, a microcontroller, a microprocessor, a computational system, or the like, or any other module or component or module of a computational system to encode thereon computer-executable instructions, software programs, and/or other data. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), virtual or physical computer system memory, physical memory hardware such as random access memory (such as, DRAM, SRAM, EDO RAM), and so forth. Although not depicted, any of the devices or components described herein may include a computer-readable medium or other memory for storing program instructions, data, and the like.

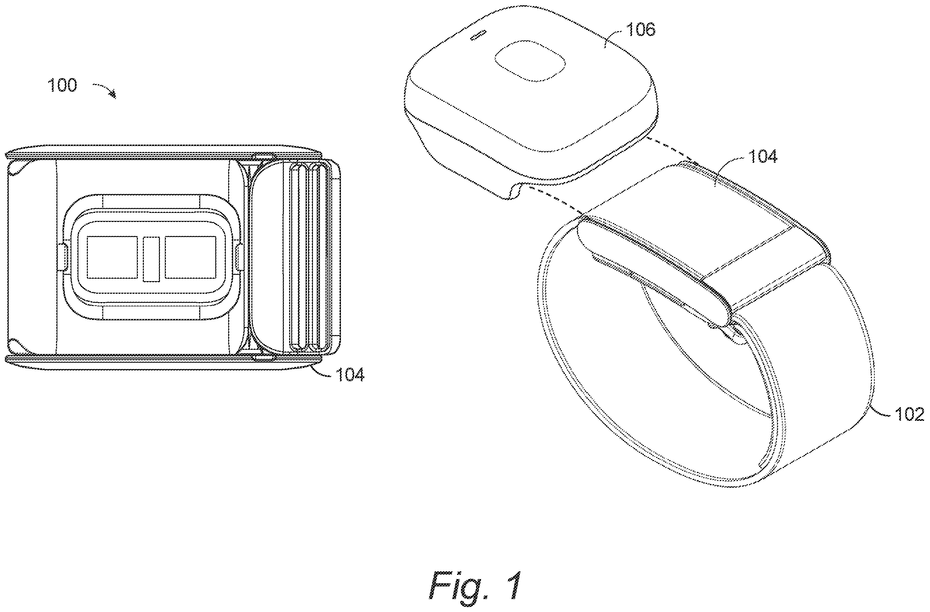

Fig. 1 shows a physiological monitoring system. The system 100 may include a wearable monitor 104 that is configured for physiological monitoring. The system 100 may also include a removable and replaceable battery 106 for recharging the wearable monitor 104. The wearable monitor 104 may include a strap 102 or other retaining system(s) for securing the wearable monitor 104 in a position on a wearer's body for the acquisition of physiological data as described herein. For example, the strap 102 may include a slim elastic band formed of any suitable elastic material such as a rubber or a woven polymer fiber such as a woven polyester, polypropylene, nylon, spandex, and so forth. The strap 102 may be adjustable to accommodate different wrist sizes, and may include any latches, hasps, or the like to secure the wearable monitor 104 in an intended position for monitoring a physiological signal. While a wrist-worn device is depicted, it will be understood that the wearable monitor 104 may be configured for positioning in any suitable location on a user's body, based on the sensing modality and the nature of the signal to be acquired. For example, the wearable monitor 104 may be configured for use on a wrist, an ankle, a bicep, a chest, or any other suitable location(s), and the strap 102 may be, or may include, a waistband or other elastic band or the like within an article of clothing or accessory. The wearable monitor 104 may also or instead be structurally configured for placement on or within a garment, e.g., permanently or in a removable and replaceable manner. To that end, the wearable monitor 104 may be shaped and sized for placement within a pocket, slot, and/or other housing that is coupled to or embedded within a garment. In such configurations, the pocket or other retaining arrangement on the garment may include sensing windows or the like so that the wearable monitor 104 can operate while placed for use in the garment. United States Pat. No. 11,185,292 describes non-limiting example embodiments of suitable wearable monitors 104.

The system 100 may include any hardware components, subsystems, and the like to support various functions of the wearable monitor 104 such as data collection, processing, display, and communications with external resources. For example, the system 100 may include hardware for a heart rate monitor using, e.g., photoplethysmography, electrocardiography, or any other technique(s). The system 100 may be configured such that, when the wearable monitor 104 is placed for use about a wrist (or at some other body location), the system 100 initiates acquisition of physiological data from the wearer. In some embodiments, the pulse or heart rate may be acquired optically based on a light source (such as light emitting diodes (LEDs)) and optical detectors in the wearable monitor 104. The LEDs may be positioned to direct illumination toward the user's skin, and optical detectors such as photodiodes may be used to capture illumination intensity measurements indicative of illumination from the LEDs that is reflected and/or transmitted by the wearer's skin.

The system 100 may be configured to record other physiological and/or biomechanical parameters including, but not limited to, skin temperature (using a thermometer), galvanic skin response (using a galvanic skin response sensor), motion (using one or more multi-axes accelerometers and/or gyroscope), blood pressure, and the like, as well environmental or contextual parameters such as ambient light, ambient temperature, humidity, time of day, and so forth. For example, the wearable monitor 104 may include sensors such as accelerometers and/or gyroscopes for motion detection, sensors for environmental temperature sensing, sensors to measure electrodermal activity (EDA), sensors to measure galvanic skin response (GSR) sensing, and so forth. The system 100 may also or instead include other systems or subsystems supporting addition functions of the wearable monitor 104. For example, the system 100 may include communications systems to support, e.g., near field communications, proximity sensing, Bluetooth communications, Wi-Fi communications, cellular communications, satellite communications, and so forth. The wearable monitor 104 may also or instead include components such as a GeoPositioning System (GPS), a display and/or user interface, a clock and/or timer, and so forth.

The wearable monitor 104 may include one or more sources of battery power, such as a first battery within the wearable monitor 104 and a second battery 106 that is removable from and replaceable to the wearable monitor 104 in order to recharge the battery in the wearable monitor 104. Also or instead, the system 100 may include a plurality of wearable monitors 104 (and/or other physiological monitors) that can share battery power or provide power to one another. The system 100 may perform numerous functions related to continuous monitoring, such as automatically detecting when the user is asleep, awake, exercising, and so forth, and such detections may be performed locally at the wearable monitor 104 or at a remote service coupled in a communicating relationship with the wearable monitor 104 and receiving data therefrom. In general, the system 100 may support continuous, independent monitoring of a physiological signal such as a heart rate, and the underlying acquired data may be stored on the wearable monitor 104 for an extended period until it can be uploaded to a remote processing resource for more computationally complex analysis. In one aspect, the wearable monitor 104 may be a wrist-worn photoplethysmography device.

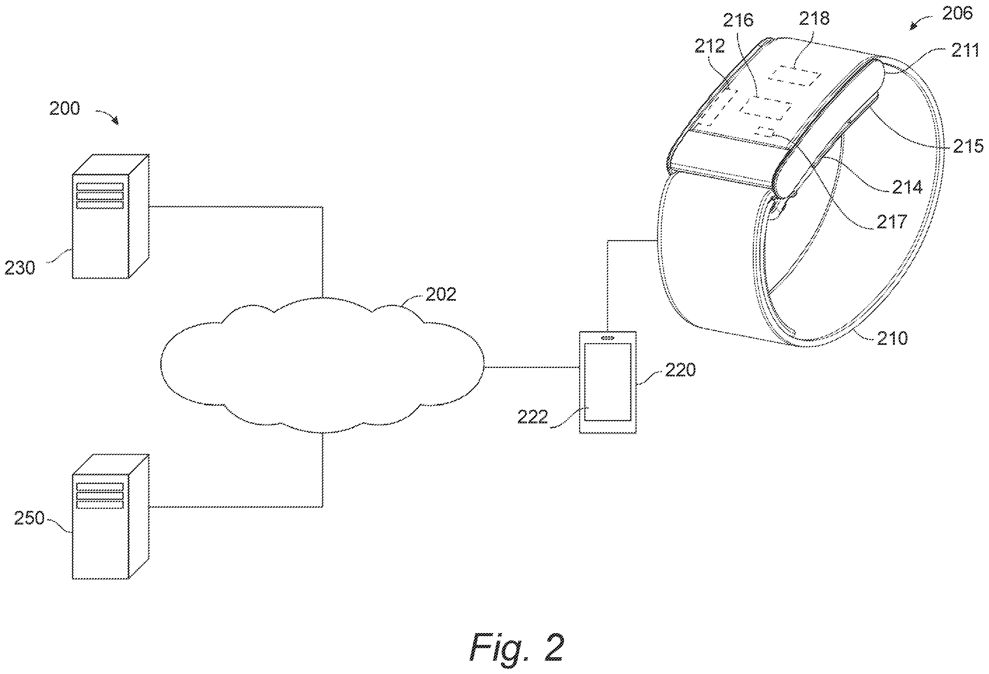

Fig. 2 illustrates a physiological monitoring system. More specifically, Fig. 2 illustrates a physiological monitoring system 200 that may be used with any of the methods or devices described herein. In general, the system 200 may include a physiological monitor 206, a user device 220, a remote server 230 with a remote data processing resource (such as any of the processors or processing resources described herein), and one or more other resources 250, all of which may be interconnected through a data network 202.

The data network 202 may be any of the data networks described herein. For example, the data network 202 may be any network(s) or internetwork(s) suitable for communicating data and information among participants in the system 200. This may include public networks such as the Internet, private networks, telecommunications networks such as the Public Switched Telephone Network or cellular networks using third generation (e.g., 3G or IMT-200), fourth generation (e.g., LTE (E-UTRA) or WiMAX-Advanced (IEEE 802.16m)), fifth generation (e.g., 5G), and/or other technologies, as well as any of a variety of corporate area or local area networks and other switches, routers, hubs, gateways, and the like that might be used to carry data among participants in the system 200. This may also include local or short-range communications infrastructure suitable, e.g., for coupling the physiological monitor 206 to the user device 220, or otherwise supporting communicating with local resources. By way of non-limiting examples, short range communications may include Wi-Fi communications, Bluetooth communications, infrared communications, near field communications, communications with RFID tags or readers, and so forth.

The physiological monitor 206 may, in general, be any physiological monitoring device or system, such as any of the wearable monitors or other monitoring devices or systems described herein. In one aspect, the physiological monitor 206 may be a wearable physiological monitor shaped and sized to be worn on a wrist or other body location. The physiological monitor 206 may include a wearable housing 211, a network interface 212, one or more sensors 214, one or more light sources 215, a processor 216, a haptic device 217 or other user input/output hardware, a memory 218, and a strap 210 for retaining the physiological monitor 206 in a desired location on a user. In one aspect, the physiological monitor 206 may be configured to acquire heart rate data and/or other physiological data from a wearer in an intermittent or substantially continuous manner. In another aspect, the physiological monitor 206 may be configured to support extended, continuous acquisition of physiological data, e.g., for several days, a week, or more.

The network interface 212 of the physiological monitor 206 may be configured to couple the physiological monitor 206 to one or more other components of the system 200 in a communicating relationship, either directly, e.g., through a cellular data connection or the like, or indirectly through a short range wireless communications channel coupling the physiological monitor 206 locally to a wireless access point, router, computer, laptop, tablet, cellular phone, or other device that can locally process data, and/or relay data from the physiological monitor 206 to the remote server 230 or other resource(s) 250 as necessary or helpful for acquiring and processing data from the physiological monitor 206.

The one or more sensors 214 may include any of the sensors described herein, or any other sensors or sub-systems suitable for physiological monitoring or supporting functions. By way of example and not limitation, the one or more sensors 214 may include one or more of a light source, an optical sensor, an accelerometer, a gyroscope, a temperature sensor, a galvanic skin response sensor, a capacitive sensor, a resistive sensor, an environmental sensor (e.g., for measuring ambient temperature, humidity, lighting, and the like), a geolocation sensor, a Global Positioning System, a proximity sensor, an RFID tag reader, and RFID tag, a temporal sensor, an electrodermal activity sensor, and the like. The one or more sensors 214 may be disposed in the wearable housing 211, or otherwise positioned and configured for physiological monitoring or other functions described herein. In one aspect, the one or more sensors 214 include a light detector configured to provide light intensity data to the processor 216 (or to the remote server 230) for calculating a heart rate and a heart rate variability. The one or more sensors 214 may also or instead include an accelerometer, gyroscope, and the like configured to provide motion data to the processor 216, e.g., for detecting activities such as a sleep state, a resting state, a waking event, exercise, and/or other user activity. In an implementation, the one or more sensors 214 may include a sensor to measure a galvanic skin response of the user. The one or more sensors 214 may also or instead include electrodes or the like for capturing electronic signals, e.g., to obtain an electrocardiogram and/or other electrically-derived physiological measurements.

The processor 216 and memory 218 may be any of the processors and memories described herein. In one aspect, the memory 218 may store physiological data obtained by monitoring a user with the one or more sensors 214, and or any other sensor data, program data, or other data useful for operation of the physiological monitor 206 or other components of the system 200. It will be understood that, while only the memory 218 on the physiological monitor is illustrated, any other device(s) or components of the system 200 may also or instead include a memory to store program instructions, raw data, processed data, user inputs, and so forth. In one aspect, the processor 216 of the physiological monitor 206 may be configured to obtain heart rate data from the user, such as heart rate data including or based on the raw data from the sensors 214. The processor 216 may also or instead be configured to determine, or assist in a determination of, a condition of the user related to, e.g., health, fitness, strain, recovery sleep, or any of the other conditions described herein.

The one or more light sources 215 may be coupled to the wearable housing 211 and controlled by the processor 216. At least one of the light sources 215 may be directed toward the skin of a user adjacent to the wearable housing 211. Light from the light source 215, or more generally, light at one or more wavelengths of the light source 215, may be detected by one or more of the sensors 214, and processed by the processor 216 as described herein.

The system 200 may further include a remote data processing resource executing on a remote server 230. The remote data processing resource may include any of the processors and related hardware described herein, and may be configured to receive data transmitted from the memory 218 of the physiological monitor 206, and to process the data to detect or infer physiological signals of interest such as heart rate, heart rate variability, respiratory rate, pulse oxygen, blood pressure, and so forth. The remote server 230 may also or instead evaluate a condition of the user such as a recovery state, sleep state, exercise activity, exercise type, sleep quality, daily activity strain, and any other health or fitness conditions that might be detected based on such data.

The system 200 may include one or more user devices 220, which may work together with the physiological monitor 206, e.g., to provide a display, or more generally, user input/output, for user data and analysis, and/or to provide a communications bridge from the network interface 212 of the physiological monitor 206 to the data network 202 and the remote server 230. For example, physiological monitor 206 may communicate locally with a user device 220, such as a smartphone of a user, via short-range communications, e.g., Bluetooth, or the like, for the exchange of data between the physiological monitor 206 and the user device 220, and the user device 220 may in turn communicate with the remote server 230 via the data network 202 in order to forward data from the physiological monitor 206 and to receive analysis and results from the remote server 230 for presentation to the user. In one aspect, the user device(s) 220 may support physiological monitoring by processing or pre-processing data from the physiological monitor 206 to support extraction of heart rate or heart rate variability data from raw data obtained by the physiological monitor 206. In another aspect, computationally intensive processing may advantageously be performed at the remote server 230, which may have greater memory capabilities and processing power than the physiological monitor 206 and/or the user device 220.

The user device 220 may include any suitable computing device(s) including, without limitation, a smartphone, a desktop computer, a laptop computer, a network computer, a tablet, a mobile device, a portable digital assistant, a cellular phone, a portable media or entertainment device, or any other computing devices described herein. The user device 220 may provide a user interface 222 for access to data and analysis by a user, and/or to support user control of operation of the physiological monitor 206. The user interface 222 may be maintained by one or more applications executing locally on the user device 220, or the user interface 222 may be remotely served and presented on the user device 220, e.g., from the remote server 230 or the one or more other resources 250.

In general, the remote server 230 may include data storage, a network interface, and/or other processing circuitry. The remote server 230 may process data from the physiological monitor 206 and perform physiological and/or health monitoring/analyses or any of the other analyses described herein, (e.g., analyzing sleep, determining strain, assessing recovery, and so on), and may host a user interface for remote access to this data, e.g., from the user device 220. The remote server 230 may include a web server or other programmatic front end that facilitates web-based access by the user devices 220 or the physiological monitor 206 to the capabilities of the remote server 230 or other components of the system 200.

The system 200 may include other resources 250, such as any resources that can be usefully employed in the devices, systems, and methods as described herein. For example, these other resources 250 may include other data networks, databases, processing resources, cloud data storage, data mining tools, computational tools, data monitoring tools, algorithms, and so forth. In another aspect, the other resources 250 may include one or more administrative or programmatic interfaces for human actors such as programmers, researchers, annotators, editors, analysts, coaches, and so forth, to interact with any of the foregoing. The other resources 250 may also or instead include any other software or hardware resources that may be usefully employed in the networked applications as contemplated herein. For example, the other resources 250 may include payment processing servers or platforms used to authorize payment for access, content, or option/feature purchases. In another aspect, the other resources 250 may include certificate servers or other security resources for third-party verification of identity, encryption or decryption of data, and so forth. In another aspect, the other resources 250 may include a desktop computer or the like co-located (e.g., on the same local area network with, or directly coupled to through a serial or USB cable) with a user device 220, wearable strap 210, or remote server 230. In this case, the other resources 250 may provide supplemental functions for components of the system 200 such as firmware upgrades, user interfaces, and storage and/or pre-processing of data from the physiological monitor 206 before transmission to the remote server 230.

The other resources 250 may also or instead include one or more web servers that provide web-based access to and from any of the other participants in the system 200. While depicted as a separate network entity, it will be readily appreciated that the other resources 250 (e.g., a web server) may also or instead be logically and/or physically associated with one of the other devices described herein, and may for example, include or provide a user interface 222 for web access to the remote server 230 or a database or other resource(s) to facilitate user interaction through the data network 202, e.g., from the physiological monitor 206 or the user device 220.

In another aspect, the other resources 250 may include fitness equipment or other fitness infrastructure. For example, a strength training machine may automatically record repetitions and/or added weight during repetitions, which may be wirelessly accessible by the physiological monitor 206 or some other user device 220. More generally, a gym may be configured to track user movement from machine to machine, and report activity from each machine in order to track various strength training activities in a workout. The other resources 250 may also or instead include other monitoring equipment or infrastructure. For example, the system 200 may include one or more cameras to track motion of free weights and/or the body position of the user during repetitions of a strength training activity or the like. Similarly, a user may wear, or have embedded in clothing, tracking fiducials such as visually distinguishable objects for image-based tracking, or radio beacons or the like for other tracking. In another aspect, weights may themselves be instrumented, e.g., with sensors to record and communicated detected motion, and/or beacons or the like to self-identify type, weight, and so forth, in order to facilitate automated detection and tracking of exercise activity with other connected devices.

One limitation on wearable sensors can be body placement. Devices are typically wrist-based, and may occupy a location that a user would prefer to reserve for other devices or jewelry, or that a user would prefer to leave unadorned for aesthetic or functional reasons. This location also places constraints on what measurements can be taken, and may also limit user activities. For example, a user may be prevented from wearing boxing gloves while wearing a sensing device on their wrist. To address this issue, physiological monitors may also or instead be embedded in clothing, which may be specifically adapted for physiological monitoring with the addition of communications interfaces, power supplies, device location sensors, environmental sensors, geolocation hardware, payment processing systems, and any other components to provide infrastructure and augmentation for wearable physiological monitors. Such "smart garments" offer additional space on a user's body for supporting monitoring hardware, and may further enable sensing techniques that cannot be achieved with single sensing devices. For example, embedding a plurality of physiological sensors or other electronic/communication devices in a shirt may allow electrocardiogram (ECG) based heart rate measurements to be gathered from a torso region of the wearer; wireless antennas to be placed above the upper portion of the thoracic spine to achieve desired communications signals; a contactless payment system to be embedded in a sleeve cuff for interactions with a payment terminal; and muscle oxygen saturation measurements to be gathered from muscles such as the pectoralis major, latissimus dorsi, biceps brachii, and other major muscle groups. This non-exhaustive list illustrates just some examples of technology that may be incorporated into a single garment.

Smart garments may also free up body surfaces for other devices. For example, if sensors in a wrist-worn device that provide heart rate monitoring and step counting can be instead embedded in a user's undergarments, the user may still receive the biometric information they desire, while also being able to wear jewelry or other accessories for suitable occasions.

The present disclosure generally includes smart garment systems and techniques. It will be understood that a "smart garment" as described herein generally includes a garment that incorporates infrastructure and devices to support, augment, or complement various physiological monitoring modes. Such a garment may include a wired, local communication bus for intra-garment hardware communications, a wireless communication system for intra-garment hardware communications, a wireless communication system for extra-garment communications and so forth. The garment may also or instead include a power supply, a power management system, processing hardware, data storage, and so forth, any of which may support enriched functions for the smart garment.

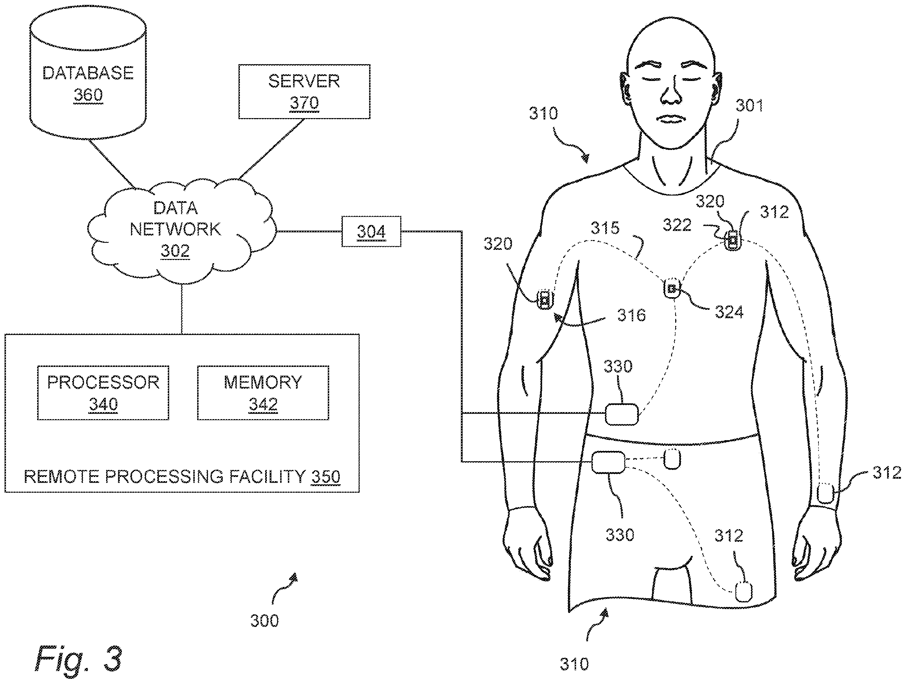

Fig. 3 shows a smart garment system. In general, the system 300 may include a plurality of components-e.g., a garment 310, one or more modules 320, a controller 330, a processor 340, a memory 342, and so on-capable of communicating with one another over a data network 302. The garment 310 may be wearable by a user 301 and configured to communicate with a module 320 having a physiological sensor 322 that is structurally configured to sense a physiological parameter of the user 301. As discussed herein, the module 320 may be controllable by the controller 330 based at least in part on a location 316 where the module 320 is located on or within the garment 310. This position-based information may be derived from an interaction and/or communication between the module 320 and the garment 310 using various techniques. It will be understood that, while two controllers 330 are shown, the garment 310 may include a single inter-garment controller, or any number of separate controllers 330 in any number of garments 310 (e.g., one per garment, or one for all garments worn by a person, etc.), and/or controllers may be integrated into other modules 320.

For communication over the data network 302, the system 300 may include a network interface 304, which may be integrated into the garment 310, included in the controller 330, or in some other module or component of the system 300, or some combination of these. The network interface 304 may generally include any combination of hardware and software configured to wirelessly communicate data to remote resources. For example, the network interface 304 may use a local connection to a laptop, smart phone, or the like that couples, in turn, to a wide area network for accessing, e.g., web-based or other network-accessible resources. The network interface 304 may also or instead be configured to couple to a local access point such as a router or wireless access point for connecting to the data network 302. In another aspect, the network interface 304 may be a cellular communications data connection for direct, wireless connection to a cellular network or the like.

The data network 302 may be any as described herein. By way of example, some embodiments of the system 300 may be configured to stream information wirelessly to a social network, a data center, a cloud service, and so forth. In some embodiments, data streamed from the system 300 to the data network 302 may be accessed by the user 301 (or other users) via a website. The network interface 304 may thus be configured such that data collected by the system 300 is streamed wirelessly to a remote processing facility 350, database 360, and/or server 370 for processing and access by the user. In some embodiments, data may be transmitted automatically, without user interactions, for example by storing data locally and transmitting the data over available local area network resources when a local access point such as a wireless access point or a relay device (such as a laptop, tablet, or smart phone) is available. In some embodiments, the system 300 may include a cellular system or other hardware for independently accessing network resources from the garment 310 without requiring local network connectivity. It will be understood that the network interface 304 may include a computing device such as a mobile phone or the like. The network interface 304 may also or instead include or be included on another component of the system 300, or some combination of these. Where battery power or communications resources can advantageously be conserved, the system 300 may preferentially use local networking resources when available, and reserve cellular communications for situations where a data storage capacity of the garment 310 is reaching capacity. Thus, for example, the garment 310 may store data locally up to some predetermined threshold for local data storage, below which data is transmitted over local networks when available. The garment 310 may also transmit data to a central resource using a cellular data network only when local storage of data exceeds the predetermined threshold.

The garment 310 may include one or more designated areas 312 for positioning a module to sense a physiological parameter of the user 301 wearing the garment 310. One or more of the designated areas 312 may be specifically tailored for receiving a module 320 therein or thereon. For example, a designated area 312 may include a pocket structurally configured to receive a module 320 therein. Also or instead, a designated area 312 may include a first fastener configured to cooperate with a second fastener disposed on a module 320. One or more of the first fastener and the second fastener may include at least one of a hook-and-loop fastener, a button, a clamp, a clip, a snap, a projection, and a void.

By placing a pocket or the like in one of these designated areas 312, a position of a module 320 can be controlled, and where an RFID tag, sensor, or the like is used, the designated area 312 can specifically sense when a module 320 is positioned there for monitoring, and can communicate the detected location to any suitable control circuitry.

The garment 310 may also or instead incorporate other infrastructure 315 to cooperate with a module 320. For example, the garment infrastructure 315 may include infrastructure 315 related to ECG devices, such as ECG pads (or otherwise electrically conductive sensor pads and/or electrodes that connect to the module 320, controller 330, and/or another component of the system 300), lead wires, and the like. By way of further example, the garment infrastructure 315 may include wires or the like embedded in the garment 310 to facilitate wired data or power transfer between installed modules 320 and other system components (including other modules 320). The infrastructure 315 may also or instead include integrated features for, e.g., powering modules, supporting data communications among modules, and otherwise supporting operation of the system 300. The infrastructure 314 may also or instead include location or identification tags or hardware, a power supply for powering modules 320 or other hardware, communications infrastructure as described herein, a wired intra-garment network, or supplemental components such as a processor, a Global Positioning System (GPS), a timing device, e.g., for synchronizing signals from multiple garments, a beacon for synchronizing signals among multiple modules 320, and so forth. More generally, any hardware, software, or combination of these suitable for augmenting operation of the garment 310 and a physiological monitoring system using the garment 310 may be incorporated as infrastructure 315 into the garment 310 as contemplated herein.

The modules 320 may generally be sized and shaped for placement on or within the one or more designated areas 312 of the garment 310. For example, in certain implementations, one or more of the modules 320 may be permanently affixed on or within the garment 310. In such instances, the modules 320 may be washable. Also or instead, in certain implementations, one or more of the modules 320 may be removable and replaceable relative to the garment 310. In such instances, the modules 320 need not be washable, although a module 320 may be designed to be washable and/or otherwise durable enough to withstand a prolonged period of engagement with a designated area 312 of the garment 310. A module 320 may be capable of being positioned in more than one of the designated areas 312 of the garment 310. That is, one or more of the plurality of modules 320 may be configured to sense data using a physiological sensor 322 in a plurality of designated areas 312 of the garment 310.

A module 320 may include one or more physiological sensors 322 and a communications interface 324 programmed to transmit data from at least one of the physiological sensors 322. For example, the physiological sensors 322 may include one or more of a heart rate monitor (e.g., one or more PPG sensors or the like), an oxygen monitor (e.g., a pulse oximeter), a blood pressure monitor, a thermometer, an accelerometer, a gyroscope, a position sensor, a Global Positioning System, a clock, a galvanic skin response (GSR) sensor, or any other electrical, acoustic, optical, or other sensor or combination of sensors and the like useful for physiological monitoring, environmental monitoring, or other monitoring as described herein. In one aspect, the physiological sensors 322 may include a conductivity sensor or the like used for electromyography, electrocardiography, electroencephalography, or other physiological sensing based on electrical signals. The data received from the physiological sensors 322 may include at least one of heart rate data and/or similar data related to blood flow (e.g., from PPG sensors), muscle oxygen saturation data, temperature data, movement data, position/location data, environmental data, temporal data, blood pressure data, and so on.

Thus, certain embodiments include one or more physiological sensors 322 configured to provide continuous measurements of heart rate using photoplethysmography or the like. The physiological sensor 322 may include one or more light emitters for emitting light at one or more desired frequencies toward the user's skin, and one or more light detectors for received light reflected from the user's skin. The light detectors may include a photo-resistor, a phototransistor, a photodiode, and the like. A processor may process optical data from the light detector(s) to calculate a heart rate based on the measured, reflected light. The optical data may be combined with data from one or more motion sensors, e.g., accelerometers and/or gyroscopes, to minimize or eliminate noise in the heart rate signal caused by motion or other artifacts. The physiological sensor 322 may also or instead provide at least one of continuous motion detection, environmental temperature sensing, electrodermal activity (EDA) sensing, galvanic skin response (GSR) sensing, and the like.

The system 300 may include different types of modules 320. For example, a number of different modules 320 may each provide a particular function. Thus, the garment 310 may house one or more of a temperature module, a heart rate/PPG module, a muscle oxygen saturation module, a haptic module, a wireless communication module, or combinations thereof, any of which may be integrated into a single module 320 or deployed in separate modules 320 that can communicate with one another. Some measurements such as temperature, motion, optical heart rate detection, and the like, may have preferred or fixed locations, and pockets or fixtures within the garment 310 may be adapted to receive specific types of modules 320 at specific locations within the garment 310. For example, motion may preferentially be detected at or near extremities while heart rate data may preferentially be gathered near major arteries. In another aspect, some measurements such as temperature may be measured anywhere, but may preferably be measured at a single location in order to avoid certain calibration issues that might otherwise arise through arbitrary placement.

In another aspect, the system 300 may include two or more modules 320 placed at different locations and configured to perform differential signal analysis. For example, the rate of pulse travel and the degree of attenuation in a cardiac signal may be detected using two or more modules at two or more locations, e.g., at the bicep and wrist of a user, or at other locations similarly positioned along an artery. These multiple measurements support a differential analysis that permits useful inferences about heart strength, pliability of circulatory pathways, blood pressure, and other aspects of the cardiovascular system that may indicate cardiac age, cardiac health, cardiac conditions, and so forth. Similarly, muscle activity detection might be measured at different locations to facilitate a differential analysis for identifying activity types, determining muscular fitness, and so forth. More generally, multiple sensors can facilitate differential analysis. To facilitate this type of analysis with greater precision, the garment infrastructure may include a beacon or clock for synchronizing signals among multiple modules, particularly where data is temporarily stored locally at each module, or where the data is transmitted to a processor from different locations wirelessly where packet loss, latency, and the like may present challenges to real time processing.

The communications interface 324 may be any as described herein, for example including any of the features of the network interface 304 described above.

The controller 330 may be configured, e.g., by computer executable code or the like, to determine a location of the module 320. This may be based on contextual measurements such as accelerometer data from the module 320, which may be analyzed by a machine learning model or the like to infer a body position. In another aspect, this may be based on other signals from the module 320. For example, signals from sensors such as photodiodes, temperature sensors, resistors, capacitors, and the like may be used alone or in combination to infer a body position. In another aspect, the location may be determined based on a proximity of a module 320 to a proximity sensor, RFID tag, or the like at or near one of the designated areas 312 of the garment 310. Based on the location, the controller 330 may adapt operation of the module 320 for location-specific operation. This may include selecting filters, processing models, physiological signal detections, and the like. It will be understood that operations of the controller 330, which may be any controller, microcontroller, microprocessor, or other processing circuitry, or the like, may be performed in cooperation with another component of the system 300 such as the processor 340 described herein, one or more of the modules 320, or another computing device. It will also be understood that the controller 330 may be located on a local component of the system 300 (e.g., on the garment 310, in a module 320, and so on) or as part of a remote processing facility 350, or some combination of these. Thus, in an aspect, a controller 330 is included in at least one of the plurality of modules 320. And, in another aspect, the controller 330 is a separate component of the garment 310, and serves to integrate functions of the various modules 320 connected thereto. The controller 330 may also or instead be remote relative to each of the plurality of modules 320, or some combination of these.

The controller 330 may be configured to control one or more of (i) sensing performed by a physiological sensor 322 of the module 320 and (ii) processing by the module 320 of the data received from a physiological sensor 322. That is, in certain aspects, the combination of sensors in the module 320 may vary based on where it is intended to be located on a garment 310. In another aspect, processing of data from a module 320 may vary based on where it is located on a garment 310. In this latter aspect, a processing resource such as the controller 330 or some other local or remote processing resource coupled to the module 320 may detect the location and adapt processing of data from the module 320 based on the location. This may, for example, include a selection of different models, algorithms, or parameters for processing sensed data.

In another aspect, this may include selecting from among a variety of different activity recognition models based on the detected location. For example, a variety of different activity recognition models may be developed such as machine learning models, lookup tables, analytical models, or the like, which may be applied to accelerometer data to detect an activity type. Other motion data such as gyroscope data may also or instead be used, and activity recognition processes may also be augmented by other potentially relevant data such as data from a barometer, magnetometer, GPS system, and so forth. This may generally discriminate, e.g., between being asleep, at rest, or in motion, or this may discriminate more finely among different types of athletic activity such as walking, running, biking, swimming, playing tennis, playing squash, and so forth. While useful models may be developed for detecting activities in this manner, the nature of the detection will depend upon where the accelerometers are located on a body. Thus, a processing resource may usefully identify location first using location detection systems (such as tags, electromechanical bus connections, etc.) built into the garment 310, and then use this detected location to select a suitable model for activity recognition. This technique may similarly be applied to calibration models, physiological signals processing models, and the like, or to otherwise adapt processing of signals from a module 320 based on the location of the module 320. In general, determining a location of a module 320 may include, e.g., receiving a sensed location for the module 320, determining the location based on communications between the module 320 and the garment 310, determining the location based on data received from a physiological sensor 322 of the module 320, and so forth.

Once determined using any of the techniques above, the location of a module 320 may be transmitted for storage and analysis to a remote processing facility 350, a database 360, or the like. That is, in addition to the module 320 using this information locally to configure itself for the location in which it is worn, the module 320 may communicate this information to other modules 320, peripherals, or the cloud. Processing this information in the cloud may help an organization determine if a module 320 has ever been installed on a garment 310, which locations are most used, and how modules 320 perform differently in different locations. These analytics may be useful for many purposes, and may, for example, be used to improve the design or use of modules 320 and garments 310, either for a population, for a user type, or for a particular user.

As stated above, the system 300 may further include a processor 340 and a memory 342. In general, the memory 342 may bear computer executable code configured to be executed by the processor 340 to perform processing of the data received from one or more modules 320. One or more of the processor 340 and the memory 342 may be located on a local component of the system 300 (e.g., the garment 310, a module 320, the controller 330, and the like) or as part of a remote processing facility 350 or the like as shown in the figure. Thus, in an aspect, one or more of the processor 340 and the memory 342 is included on at least one of the plurality of modules 320. In this manner, processing may be performed on a central module, or on each module 320 independently. In another aspect, one or more of the processor 340 and the memory 342 is remote relative to each of the plurality of modules 320. For example, processing may be performed on a connected peripheral device such as smart phone, laptop, local computer, or cloud resource.

The processor 340 may be configured to assess the quality of the data received from a physiological sensor 322 of the module 320, otherwise process data as described herein. The memory 342 may store one or more algorithms, models, and supporting data (e.g., parameters, calibration results, user selections, and so forth) and the like for transforming data received from a physiological sensor 322 of the module 320. In this manner, suitable models, algorithms, tuning parameters, and the like may be selected for use in transforming the data based on the location of the module 320 as determined by the controller 330 and/or processor 340 as described herein.

A database 360 may be located remotely and in communication with the system 300 via the data network 302. The database 360 may store data related to the system 300 such as any discussed herein-e.g., sensed data, processed data, transformed data, metadata, physiological signal processing models and algorithms, personal activity history, and the like. The system 300 may further include one or more servers 370 that host data, provide a user interface, process data, and so forth in order to facilitate use of the modules 320 and garments 310 as described herein.

It will be appreciated that the garment 310, modules 320, and accompanying garment infrastructure and remote networking/processing resources, may advantageously be used in combination to improve physiological monitoring and achieve modes of monitoring not previously available.

One or more of the devices and systems described herein may include circuitry for both wireless charging and wireless data transmission, e.g., where the corresponding circuits can operate independently from one another, and where the corresponding antennae are located proximal to one another (for instance, the circuitry for wireless charging and the circuitry for wireless data transmission may include separate coils disposed substantially along the same plane, or otherwise in relative close proximity in a device or system). In such aspects, one or more measures may be taken so that a wireless data transfer process does not interfere with a wireless power transfer process, more specifically by coupling the data circuitry into the electromagnetic field for the wireless power transfer in a manner that alters the resonant frequency or otherwise destructively interferes with power transfer, thereby decreasing efficiency when charging a device. For example, a switch may be included to disable circuitry for data transmission when certain wireless charging activity is present, thereby allowing for relatively unimpeded and efficient wireless charging of a device. The switch may also be operable to enable operation of data transmission circuitry when certain wireless charging activity is not present, and/or in other circumstances where data transmission is desirable.

Thus, for example, in the context of a physiological monitor, such as any of those described herein, the physiological monitor may include both a wireless power receiver (or similar) and a wireless data tag reader (or similar). In general, these sub-systems may conform to one or more Near Field Communication (NFC) specifications for protocols and physical architectures, or any other standards suitable for wireless power and data transmission. The power circuitry may be used, e.g., to charge a battery on the physiological monitor so that the device can be recharged without physically connecting to a power source. The data circuitry may be used, e.g., as a wireless data tag reader or the like to read data from nearby data sources such as identification tags in user apparel and the like, and/or for connecting the physiological monitor to other accessories, e.g., a watch face or GPS unit that may be physically coupled to the physiological monitor. In general, the physiological monitor may include separate circuity (separate coils) for these wireless power and data systems, such as separate processing circuitry and/or separate antennae. The antennae may be disposed substantially along the same plane of the physiological monitor (e.g., with one coil disposed substantially inside or adjacent to the other). In one aspect, the antennae may be in parallel planes, however, it will be noted that distance tolerances for NFC standard devices are relatively small, and the physically housing for these antennae will preferably enforce an identical or substantially identical distance for both antennae in such architectures. In this context, the positions of the antennae may be as close to parallel as possible within reasonable manufacturing tolerances, or as close to parallel as possible when disposed on two different layers of a shared printed circuit board, or preferably, when disposed on a single layer of a shared printed circuit board. The physiological monitor may further include a switch (e.g., a radio frequency (RF) switch or the like) in-line with the coil for the wireless data tag reader to disable the wireless data tag reader when power is being received to mitigate any effects on the efficiency of the wireless power transfer process. In particular, the switch may be configured to open when power is being received, and may be configured to close when the physiological monitor is looking for data tag to read.

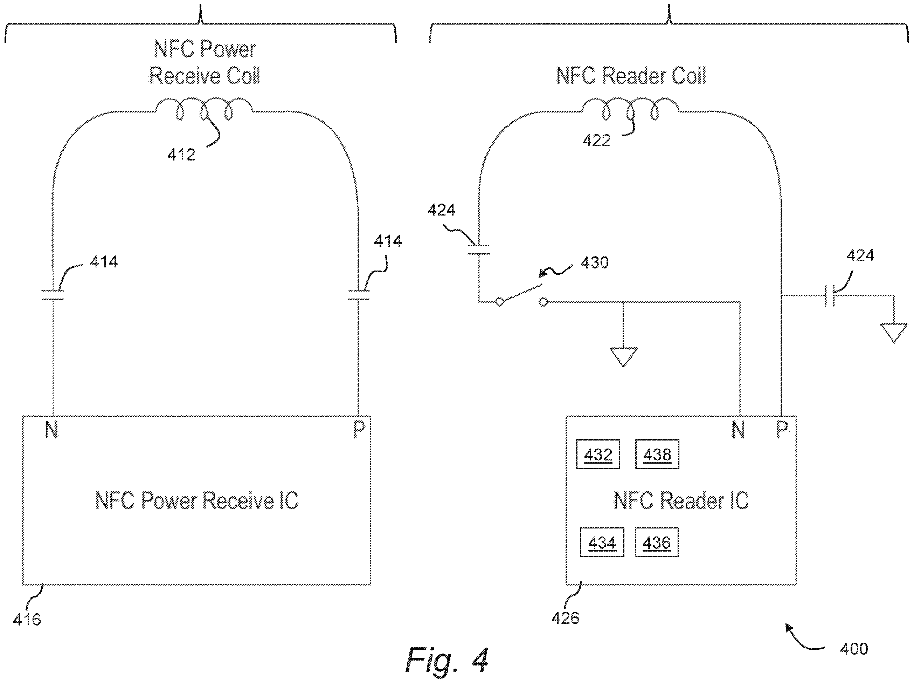

Fig. 4 illustrates circuitry for wireless power and data transmission. It will be understood that the system 400 shown in Fig. 4 may be present in any of the devices, systems, and methods described herein. Thus, for example, the system 400 may be embodied in a device further including a physiological monitor such as any of the physiological monitoring systems and devices described herein. The circuitry includes a first circuit 410 configured to receive wireless power from a wireless charging device, and a second circuit 420 configured for wireless data transmission (e.g., configured to wirelessly receive and/or read data from a data tag or the like, and/or to send data as appropriate). It will be understood that, although shown as separate circuits, as described herein, the first circuit 410 and the second circuit 420 may be disposed substantially along the same plane or stacked on two adjacent planes within a device or system, and/or they may be located in relatively close proximity to one another, and/or they may share certain passive, active, or programmable electronic components, e.g., as necessary or helpful for conserving space within a device.

The first circuit 410 includes a power antenna 412 (shown as an inductor coil in the circuit diagram, and labeled as "NFC Power Receive Coil"), and may include one or more tuning passives 414 (shown as capacitors in the circuit diagram, although they may include any combination of passive components suitable for radio frequency (RF) tuning of the first circuit 410) such as discrete or physically tunable passive components for fine tuning an RF resonance of the first circuit 410. The system also includes a wireless power receiver 416 (e.g., an integrated circuit such as an NFC Power Receive IC chip or the like) that converts power received through the power antenna 412 into electrical power for use by a device (e.g., as the load in an electrical power circuit). Thus, a device or system includes a first circuit 410 with a power antenna 412 and a wireless power receiver 416 coupled to the power antenna 412. The power antenna 412 may be a planar antenna such as a planar coil antenna or any other antenna or antennae suitable for receiving power from a matched power transmission system or the like.

The second circuit 420 includes a data antenna 422 (shown as an inductor coil in the circuit diagram, and labeled as "NFC Reader Coil"), and may include one or more tuning passives 424 (shown as capacitors in the circuit diagram, although they may include any combination of passive components suitable for RF tuning of the second circuit 420) such as discrete or physically tunable passive components for fine tuning an RF resonance of the second circuit 420. The system also includes a wireless data receiver 426 (e.g., an integrated circuit such as an NFC Reader IC chip or the like) that communicates with nearby wireless data sources and receives data, stores data, and/or provides data to one or more components of the device or system in which the second circuit 420 is present. The wireless data receiver 426 may include a processor and a memory, and/or may provide wirelessly received data to a processor and a memory of a device. Thus, a device or system generally includes a second circuit with a data antenna 422 and a wireless data receiver 426 coupled to the data antenna 422. The data antenna 422 may be a planar antenna such as a planar coil antenna, or any other antenna or antennae suitable for coupling in a communicating relationship with an external, wireless data source. And, as described herein, in aspects where the data antenna 422 is a planar coil antenna and/or the power antenna 412 is a planar coil antenna, the data antenna 422 may be coplanar or substantially coplanar with the power antenna 412 within a device or system, or in a parallel plane offset from the power antenna 412, e.g., by layers of a printed circuit board or the like. In one aspect, one or more of the antennae 412, 422 may be curved. In this case, the "plane" of one of the antennae may generally be a plane passing through or normal to a surface of the antenna.

In devices such as a wearable physiological monitor, the data antenna 422 may be positioned close to the power antenna 412 in order to conserve space. For example, the data antenna 422 may be positioned coplanar with and adjacent to the power antenna 412, or the data antenna 422 may be positioned concentrically with (or overlapping with) the power antenna 412 and offset along an axis normal to the plane of the data antenna 422, such as in a different layer of a printed circuit board that contains both antennae 412, 422, or on another printed circuit board vertically stacked with a printed circuit board containing the power antenna 412. When arranged in this manner, the two antennae 412, 422 and accompanying circuits 410, 420 may interfere with one another, decreasing the efficiency of radio frequency (RF) transmissions to and from the device. In this context, one of the antennae 412, 422, such as the data antenna 422, may advantageously be decoupled while receiving power in order to mitigate detuning or other RF interference with the other one of the antennae 412, 422. While this is generally useful for either antenna, the advantages of decoupling an antenna to mitigate RF circuit interference may provide the most benefit in high power applications, such as power transfer for battery charging or the like.

To this end, the second circuit 420 includes a switch 430 configured to selectively decouple (and couple) the data antenna 422 to the wireless data receiver 426. The switch 430 is controllable via control circuitry 432, which may be integrated into the wireless data receiver 426, an NFC Reader IC or other chip or control circuitry, or embodied in an additional processor, controller, microcontroller, or other discrete and/or programmable electronics or the like that can respond to suitable control signals or operate independently from external control signals to selectively couple the data antenna 422 to the wireless data receiver 426 as described herein. It will be understood that in this context, coupling and decoupling does not require full, two lead coupling and decoupling. The benefits of the methods and systems described herein may be achieved with a decoupling that includes disconnecting a single terminal, e.g., with the switch 430 as illustrated in Fig. 4. Any such decoupling suitable for interrupting a continuous circuit between the terminals of the data antenna 422 and the wireless data circuitry 426 should be considered a "decoupling" (and the corresponding components should be considered "decoupled") as that term is used herein, unless otherwise specifically stated to the contrary.

Although illustrated as included with the NFC Reader IC of the wireless data receiver 427, it will be understood that components such as the control circuitry 432 may also or instead include components or circuitry separate from the NFC Reader IC and/or the wireless data receiver 426. The control circuitry 432 may include, or may be in communication with, one or more of a processor 434 and a memory 436, each of which may also be integrated into or separate from the control circuitry 432 and/or the wireless data receiver 426. In general, the processor 434 may facilitate the execution of functions according to instructions included in code contained within the memory 436. The processor 434 and the memory 436 may be any as described herein.

The control circuitry 432 is configured to connect/disconnect the data antenna 422 from the wireless data receiver 426 using the switch 430 in response to certain conditions such as conditions indicating an availability of data (or optionally, the non-availability of power). The control circuitry 432 is configured to disconnect the data antenna 422 from the wireless data receiver 427 with the switch 430 when power is being received through the power antenna 412. Similarly, the control circuitry 432 may be configured to reconnect the data antenna 422 to the wireless data receiver 426 using the switch 430 in response to certain conditions, such as when power is not being received through the power antenna 412, or when there are other indicators that a data source is available. For example, the control circuitry 432 may connect the data antenna 422 to the wireless data receiver 426 when an amount of power received by the wireless power receiver 416 is below a predetermined threshold, e.g., when the first (power) circuit 410 is not actively charging a battery or otherwise providing power to the device. The control circuitry 432 may also or instead disconnect the data antenna 422 from the wireless data receiver 426 with the switch 430 when an amount of power received by the wireless power receiver 416 is above the predetermined threshold, e.g., when the first (power) circuit 410 is actively charging a battery or otherwise providing power to the device. However, it will be understood that other events can also or instead be used as triggers that result in connecting or disconnecting the data antenna 422 from the wireless data receiver 426 via the switch 430, and that operation of the switch 430 is not limited to situations involving power transfer-e.g., a user input, a control signal indicating a pending data transmission, other logic or parameters, and the like. That is, the system 400 may, in some aspects, keep the switch 430 connected (and/or change from an open position to a closed position) when power is received, e.g., at least momentarily. According to the claimed invention, in a variety of configurations, the switch 430 more generally selectively couples the data antenna 422 to the wireless data receiver 426 in response to a data availability event. This can advantageously mitigate interference with the co-located wireless power system except when a predetermined event-a data availability event-indicates that data might be available for the system 400.

Where it is preferred or advantageous for the device to default to a connection to a power source (e.g., in the absence of power from a battery on the device), the control circuitry 432 may be configured to default to a power receive mode with the switch 430 in an open position so that the device can more efficiently receive power if/when available, more specifically by mitigating interference from the data antenna 422 and wireless data circuitry 426. In one aspect, this may be a hardware default, e.g., where the switch 430 is open in response to a power failure or other malfunction.

In another aspect, the first (power) circuit 410 may be configured to receive data, which may provide a backup or alternative data channel for the device. A variety of single antenna data/power techniques are known in the art (and in the various NFC standards), and may be suitably employed to provide a single data/power system in addition to the separate data transmission circuit. In another aspect, complementary circuitry may be provided to facilitate decoupling of the wireless power circuitry 416 from the power antenna 412 during data transmissions, although less overall power savings will be achieved, and this may require additional management of switch control circuitry to ensure that the wireless power circuitry 416 is available, e.g., in the case of battery depletion and other hardware faults on a device.

According to the claimed invention, the control circuitry 432 is also configured to connect the data antenna 422 to the wireless data receiver 426 using the switch 430, and to actively ping for a readable data tag under one or more predetermined conditions. According to the claimed invention, such a predetermined condition includes detecting contact of at least part of the system with skin of a user or detection of a garment pocket around at least part of the system. Such a predetermined condition may also include passage of a time interval (e.g., periodic pinging for data), detecting motion of the device, detecting absence of power for a predetermined interval, and so forth.

For example, for a physiological monitor, a predetermined condition for making data transmissions available may include a condition when the device is placed for use against the skin of a user. For a garment-based physiological monitor, the predetermined condition includes detection of a garment pocket around at least part of the system, e.g., where the device is placed for use in a pocket or pod of a smart garment as described herein. In this context, a nearby data source (e.g., a data tag disposed on or near the pocket) may usefully provide data, e.g., to determine where on a garment (and/or a user's body) the device is located, and to adjust operation of the device according to the sensed location. For example, the device may alter operational parameters for the device (e.g., light intensity, measurement frequency, etc.) or modify a model used to calculate physiological parameters, such as by changing model parameters or selecting a location-specific model for detecting activity types, calculating physiological values, and so forth. Data requests may also or instead be used to authenticate a user, check for device compatibility, or otherwise provide useful information to the device from a nearby data source. A device or system featuring the first and second circuits 410, 420 may thus include one or more sensors 438 configured to detect one or more predetermined conditions for the control circuitry 432 to operate the switch 430 to decouple a portion of the second (data) circuit 420. And it will be understood that one or more of these sensors 438 may be configured to detect the presence or absence of a condition such as when power is being received, when power is not being received, when a device or system is placed in a position for use (e.g., based on temperature, light, capacitive touch sensing, etc.) or otherwise placed in a position of interest, when a device is moved (e.g., based on accelerometer data), when a device is connected to another device (e.g., an external battery, a charger, and/or another accessory), and so on.

While the circuits in Fig. 4 might usefully be incorporated into any system using a combination of wireless power transmission and wireless data transmission, in one aspect, the circuits depicted in Fig. 4 may be embodied in a device including a physiological monitor, such as any of the physiological monitoring devices and systems described herein. Such a physiological monitor may include a battery charged at least partially by the wireless power receiver 416. Further, the physiological monitor may include a controller (such as any as described herein, e.g., with reference to Fig. 3) configured in response to data received by the wireless data receiver 426. That is, the controller may act in response to data received by the wireless data receiver 426, where this data configures the controller to perform certain operations and/or make certain selections (e.g., selecting an appropriate model and/or parameters for sensing and/or processing physiological data). By way of example, this can include acting in response to location data received by the wireless data receiver 426 (e.g., a physiological monitor is located on the left wrist of a user, or that a physiological monitor is located within a pod or pocket of a certain portion of a smart garment, and so on), where the controller can then utilize an appropriate model, parameter, sensing operation, or otherwise for measuring physiological data at that location. In this manner, the controller may be configured to receive location data through the wireless data receiver 426 and to adjust calculations by a physiological monitoring device according to the received location data. The data received by the wireless data receiver 426 for configuring the controller may also or instead include one or more of garment type, garment size, user-specific data, and so on. Other types of data may also or instead be communicated through the wireless data receiver 426, and may be used for any of the purposes described herein.

The first circuit 410 and/or the second circuit 420 may conform to one or more NFC standards, as such standards are well-known in the art. By way of example, the wireless power receiver 416 may include an NFC power receiver or the like. Also or instead, the wireless power receiver 416 may conform to at least one NFC Forum wireless charging specification, and/or the power antenna 412 may conform to at least one NFC Forum physical specification. Similarly, the wireless data receiver 426 may include an NFC data tag reader or the like. Also or instead, the wireless data receiver 426 may conform to at least one NFC Forum data exchange specification, and/or the data antenna 422 may conform to at least one NFC Forum physical specification. More generally, the components of the system 400 may conform to any open or closed standards suitable for interaction and cooperation with other devices, power sources, data sources, and the like.

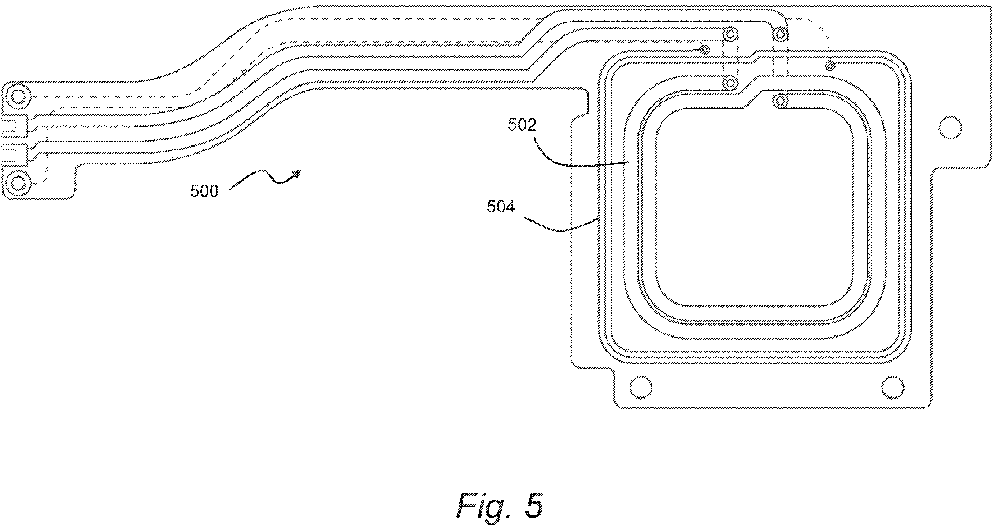

Fig. 5 shows a printed circuit board layout. In general, the printed circuit board 500 may include a first antenna 502 for wireless power transmission, such as a first planar coil antenna, planar spiral antenna, or other planar antenna or the like. The printed circuit board 500 may also include a second antenna 504 for wireless data transmission, such as a second planar coil antenna, second planar spiral antenna, or other planar antenna or the like. The first antenna 502 may be coupled to a wireless power receive circuit such as the first circuit 410 described above with reference to Fig. 4, and the second antenna 504 may be coupled to a wireless tag reader circuit such as the second circuit 420 described above with reference to Fig. 4. As illustrated in Fig. 5, the first antenna 502 and the second antenna 504 may be coplanar antennae, lying substantially along the same plane, such as a plane of a printed circuit board. However, other arrangements are also possible. For example, where the distances remain within suitable tolerances for an associated data or power transmission standard, the antennae may be positioned in different planes, such as a first layer and a second layer of a printed circuit board (provided also that the circuit board does not include any other intervening layers that might interfere with electromagnetic coupling), and may include concentric or overlapping structures along an axis normal to the plane of the printed circuit board(s) and antenna(e). Thus in one aspect, the antennae may be concentric antennae, e.g., as illustrated in Fig. 5. The antennae may also or instead include two planar antennae. The antennae may also or instead include two antennae with overlapping structures along an axis normal to the antennae, as also illustrated in Fig. 5. It will be understood that, while a printed circuit board provides a convenient substrate for NFC antennae and accompanying electronics, other packaging may also or instead be used.

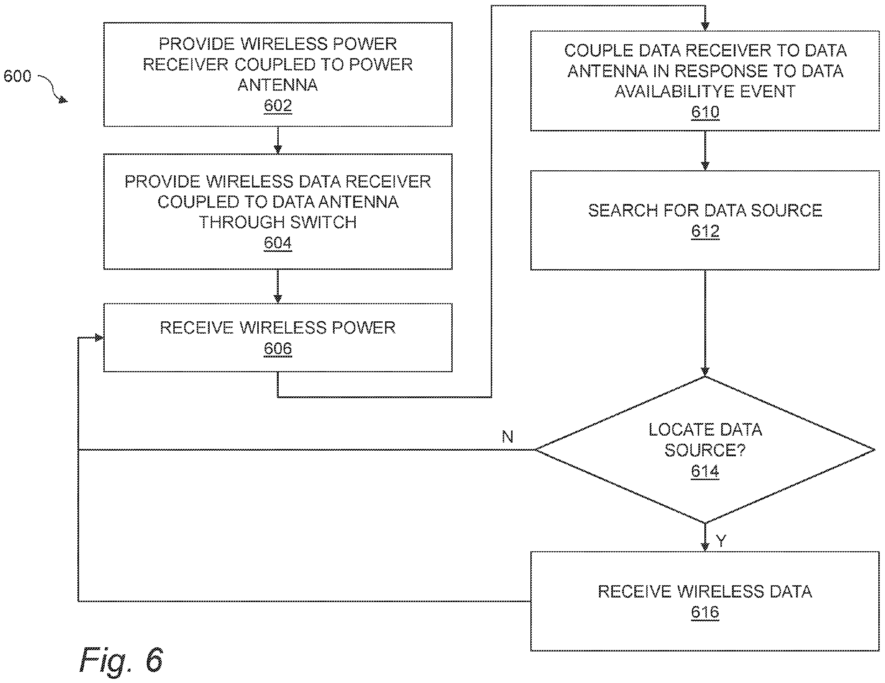

Fig. 6 is a flow chart of a method for wireless power and data transmission. It will be understood that the method 600 may be implemented using any of the devices and systems described herein. In particular, the method 600 may be implemented in a system or device featuring a physiological monitor as described herein.

As shown in step 602, the method 600 includes providing a wireless power receiver coupled to a power antenna. This may, for example, include any of the wireless power receiving circuitry and power antennae described herein. For example, the power antenna may include a planar coil power antenna.

As shown in step 604, the method 600 includes providing a wireless data receiver coupled to a data antenna through a switch. This may, for example, include any of the wireless data receiving circuitry and data antennae described herein. For example, the wireless data antenna may include a planar coil data antenna. In one aspect, the data antenna may be coplanar with the power antenna, e.g., where the data antenna and the power antenna are adjacent on a single layer of a printed circuit board. In another aspect, the data antenna may be concentric with the power antenna along a normal axis to a plane including a surface of the data antenna (or the power antenna), but vertically offset from the plane of the power antenna. For example, the data antenna may be fabricated on a different layer of a printed circuit board containing the power antenna, or on an adjacent printed circuit board, or otherwise vertically stacked with and/or overlapping the power antenna along the normal axis.

As shown in step 606, the method 600 may include receiving wireless power. As described above, this may include placing a switch that couples the wireless data receiver to the data antenna in an open position for decoupling, which may be a default operating mode for a device, even when no power is currently being wirelessly provided to the power antenna.

As shown in step 610, the method 600 includes, in response to a data availability event, operating the switch to selectively couple the wireless data receiver to the data antenna. The data availability event may include any of the data availability events described herein. For example, when an amount of power received by the wireless power receiver is below a predetermined threshold, a data availability event may occur and the method 600 may include coupling the data antenna to the wireless data circuitry and searching for a data source. The data availability event may also or instead include a detection of contact with a user or user device, or an explicit user request to transfer data. According to the claimed invention, the data availability event also or instead includes a detection of insertion into a pocket of a garment adjacent to a data tag. In another aspect, the data availability event may be a periodic event, e.g., that occurs on a predetermined schedule under control of a microprocessor or the like, even when power is generally available. More generally, the availability event may include any event that might usefully indicate a possible availability of data, and may be used as a trigger to close the switch and couple the data antenna to the wireless data receiver (or other wireless data receiving circuitry).

In some aspects, the wireless data receiver remains coupled to the data antenna unless there is a power availability event and/or another event of interest (e.g., subject to one or more other factors, logic, parameters, inputs, and the like, also or instead of a power availability event). In certain aspects, one such power availability event that can trigger operation of the switch to disconnect the data antenna from the wireless data receiver may include a predetermined amount of power being received by the wireless power receiver. Thus, selectively coupling the wireless data receiver may include leaving the wireless data receiver coupled to the data antenna when an amount of power received by the wireless power receiver is below a predetermined threshold, and/or when a wireless power source is not detected. In other aspects, the wireless data receiver may be uncoupled from the data antenna, via the switch, unless the data availability event is present, or when the data availability event expires. This can mitigate power loss due to prolonged searches for data sources, and permits resumption of efficient power transfer with minimal interference from the wireless data system.

As shown in step 612, the method 600 includes, in response to the data availability event (and the resulting physical coupling of the data receiver and antenna), searching for a data source. This includes attempting to initiate a connection to a wireless data source, such as by transmitting a ping for an NFC data tag or other nearby data source, or by otherwise monitoring an RF signal received through the data antenna for relevant signals.

As shown in step 614, the method 600 may include determining whether a data source has been located, e.g., by detecting a response to an NFC ping or other wireless query or interrogation, or by otherwise monitoring and analyzing signals received through the data antenna. When no data source is located, the method 600 may, e.g., after a predetermined number of connection attempts (e.g., pings) and/or after a predetermined amount of time, open the switch and return to a power mode, e.g., step 606, where wireless power can be (efficiently) received. It will be noted that the qualifier, "efficiently," is parenthetically added in this context because it may be possible to receive power even when the switch is closed and a wireless data circuit is active, however, the power circuit may suffer interference and degradation from concurrent operation of the data circuit. In this context, "efficiently" is intended to mean "with the interference from an adjacent data system mitigated by decoupling a data antenna from wireless data circuitry." Where a data source is located, e.g., by a response to an NFC ping, the detected data source may be interrogated, and/or data may be wirelessly received from the data source as shown in step 616. The method 600 may then return to a power mode, e.g., step 606, where wireless power can be (efficiently) received.

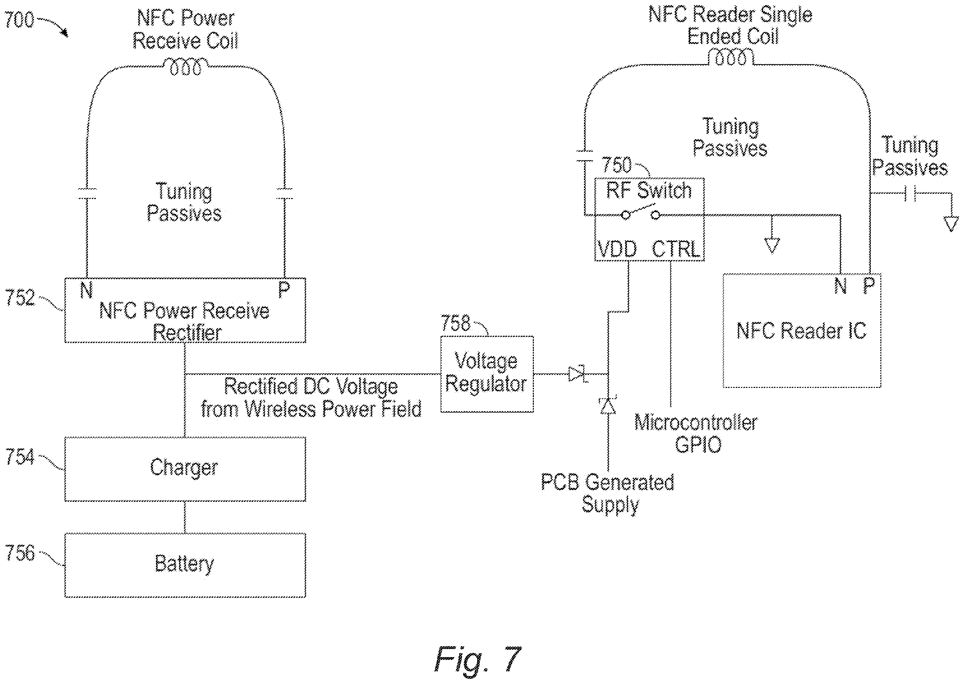

Fig. 7 illustrates circuitry for wireless power and data transmission. In general, the system 700 may be deployed on a device such as a wearable physiological monitor or other device that uses wireless power and data. The system 700 may include the antennae, wireless data circuitry, and wireless power circuitry described herein. The system may also include an NFC Power Receive Rectifier 752 or other wireless power circuitry to provide power to a charger 754 that can charge a battery 756 on a device, such as a battery environmentally sealed within a housing of the device.

In the system 700 of Fig. 7, an RF switch 750 is used to provide good (e.g., high impedance) isolation between the data antenna and the data circuitry. However, for certain RF switches, this may require additional control elements. In one aspect, a rectified DC voltage from the NFC Power Receive Rectifier 752 in the wireless power circuit may be provided to a voltage regulator 758, which may provide a regulated power output to the RF switch 750 (which may require power to maintain a known switch state). At the same time, a control input for the RF switch 750 may be grounded to provide a control signal to hold the RF switch 750 open. In response to a data availability event such as a tag detection or any of the data availability events described herein, a microcontroller or other control circuitry (not shown) in the system 700 may close the switch, more specifically by providing power from a printed circuit board or other power source on a device, and providing a logical high voltage to the control input to hold the RF switch 750 closed. It will be understood that the RF switch 750 described may additionally or alternatively be configured for different voltages at the various inputs and control to drive the RF switch 750 open or closed as desired.

More generally, the system 700, and any of the systems and devices described herein, may use any suitable combination of switches, circuits, power levels, logic values, and control systems suitable for selectively coupling and decoupling a wireless data circuit and a data antenna in response to a data availability event (or other events) as described herein.

The above systems, devices, methods, processes, and the like may be realized in hardware, software, or any combination of these suitable for the control, data acquisition, and data processing described herein. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

Thus, in one aspect, each method described above, and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared, or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above. All such permutations and combinations are intended to fall within the scope of the present disclosure.

The method steps of the implementations described herein are intended to include any suitable method of causing such method steps to be performed, consistent with the patentability of the following claims, unless a different meaning is expressly provided or otherwise clear from the context. So, for example, performing the step of X includes any suitable method for causing another party such as a remote user, a remote processing resource (e.g., a server or cloud computer) or a machine to perform the step of X. Similarly, performing steps X, Y, and Z may include any method of directing or controlling any combination of such other individuals or resources to perform steps X, Y, and Z to obtain the benefit of such steps. Thus, method steps of the implementations described herein are intended to include any suitable method of causing one or more other parties or entities to perform the steps, consistent with the patentability of the following claims, unless a different meaning is expressly provided or otherwise clear from the context. Such parties or entities need not be under the direction or control of any other party or entity and need not be located within a particular jurisdiction.

It will be appreciated that the methods and systems described above are set forth by way of example and not of limitation. Numerous variations, additions, omissions, and other modifications will be apparent to one of ordinary skill in the art. In addition, the order or presentation of method steps in the description and drawings above is not intended to require this order of performing the recited steps unless a particular order is expressly required or otherwise clear from the context. Thus, while particular embodiments have been shown and described, it will be apparent to those skilled in the art that various changes and modifications in form and details may be made therein without departing from the scope of the invention as defined by the following claims.

## Claims

1. A system comprising:
a power antenna;
a wireless power receiver coupled to the power antenna;
a data antenna;
a wireless data receiver coupled to the data antenna;
a switch selectively coupling the data antenna to the wireless data receiver; and
control circuitry configured to operate the switch to disconnect the data antenna from the wireless data receiver in response to power being received by the wireless power receiver through the power antenna,
**characterised in that** the control circuitry is further configured to respond to a data availability event, by connecting the data antenna to the wireless data receiver using the switch, and actively pinging for a readable data tag with the wireless data receiver,
wherein the data availability event includes at least one of (i) detection of contact of at least part of the system with skin of a user, or (ii) detection of a garment pocket around at least part of the system.

2. The system of claim 1, wherein the control circuitry is configured to operate the switch to connect the data antenna to the wireless data receiver when an amount of power received by the wireless power receiver is below a predetermined threshold.

3. The system of any of the preceding claims, wherein the control circuitry is configured to default to a power receive mode with the switch in an open position.

4. The system of any of the preceding claims, further comprising a physiological monitor.

5. The system of claim 4, wherein the physiological monitor includes a battery charged at least partially by the wireless power receiver.

6. The system of any of claims 4 to 5, wherein the physiological monitor includes a controller configured to operate the physiological monitor in response to data received by the wireless data receiver.

7. The system of claim 6, wherein the data includes at least one of a garment type and a user's body location.

8. The system of any of the preceding claims, wherein the power antenna is a planar coil antenna.

9. The system of any of the preceding claims, wherein the data antenna is a planar coil antenna.

10. The system of any of claims 8 to 9, wherein the data antenna is in a parallel plane to the power antenna.

11. The system of any of claims 8 to 9, wherein the data antenna overlaps the power antenna along an axis normal to a plane of the data antenna.

12. The system of any of claims 8 to 10, wherein the data antenna is concentric with the power antenna.

13. The system of any of the preceding claims, wherein the wireless power receiver includes a Near Field Communication power receiver and the wireless data receiver includes a Near Field Communication data tag reader.

14. A method comprising:
detecting a data availability event on a device, wherein:
the data availability event indicates an availability of data wirelessly available for the device,
the device includes a wireless power receiver coupled to a power antenna,
the device includes a wireless data receiver coupled to a data antenna, and
the device includes a switch selectively coupling the wireless data receiver to the data antenna;
in response to detecting the data availability event, operating the switch to selectively couple the wireless data receiver to the data antenna; and
initiating a connection between the device and a wireless data source through the data antenna,
the method **characterised in that**
the data availability event includes detection of insertion of the device into a pocket of a garment adjacent to a data tag.

15. The method of claim 14, wherein the data availability event further includes a detection of contact with the skin of a user

## Patentansprüche

1. Ein System, das Folgendes umfasst:
eine Leistungsantenne;
einen drahtlosen Leistungsempfänger, der mit der Leistungsantenne gekoppelt ist;
eine Datenantenne;
einen drahtlosen Datenempfänger, der mit der Datenantenne gekoppelt ist;
einen Schalter, der die Datenantenne selektiv mit dem drahtlosen Datenempfänger koppelt; und
eine Steuerschaltung, die so konfiguriert ist, dass sie den Schalter betätigt, um die Datenantenne vom drahtlosen Datenempfänger zu trennen, wenn der drahtlose Leistungsempfänger über die Leistungsantenne Leistung empfängt,
**dadurch gekennzeichnet, dass**
die Steuerschaltung ferner so konfiguriert ist, dass sie auf ein Datenverfügbarkeitsereignis reagiert, indem sie die Datenantenne unter Verwendung des Schalters mit dem drahtlosen Datenempfänger verbindet und aktiv nach einem lesbaren Datenetikett mit dem drahtlosen Datenempfänger sucht,
wobei das Datenverfügbarkeitsereignis mindestens eines umfasst von (i) Erkennen eines Kontakts mindestens eines Teils des Systems mit der Haut eines Benutzers oder (ii) Erkennen einer Kleidungsstücktasche um mindestens einen Teil des Systems.

2. System nach Anspruch 1, wobei die Steuerschaltung so konfiguriert ist, dass sie den Schalter betätigt, um die Datenantenne mit dem drahtlosen Datenempfänger zu verbinden, wenn eine von dem drahtlosen Energieempfänger empfangene Energiemenge unter einem vorbestimmten Schwellenwert liegt.

3. System nach einem der vorhergehenden Ansprüche, wobei die Steuerschaltung so konfiguriert ist, dass sie standardmäßig in einen Energieempfangsmodus wechselt, wenn sich der Schalter in einer offenen Position befindet.

4. System nach einem der vorhergehenden Ansprüche, das ferner einen physiologischen Monitor umfasst.

5. Das System nach Anspruch 4, wobei der physiologische Monitor eine Batterie umfasst, die zumindest teilweise durch den drahtlosen Energieempfänger geladen wird.

6. Das System nach einem der Ansprüche 4 bis 5, wobei der physiologische Monitor einen Controller umfasst, der so konfiguriert ist, dass er den physiologischen Monitor als Reaktion auf Daten betreibt, die durch den drahtlosen Datenempfänger empfangen werden.

7. Das System nach Anspruch 6, wobei die Daten mindestens einen Kleidungsstücktyp und/oder eine Körperstelle eines Benutzers umfassen.

8. System nach einem der vorhergehenden Ansprüche, wobei die Leistungsantenne eine Flachspulenantenne ist.

9. System nach einem der vorhergehenden Ansprüche, wobei die Datenantenne eine Flachspulenantenne ist.

10. System nach einem der Ansprüche 8 bis 9, wobei die Datenantenne in einer parallelen Ebene zur Leistungsantenne liegt.

11. System nach einem der Ansprüche 8 bis 9, wobei die Datenantenne die Leistungsantenne entlang einer Achse senkrecht zu einer Ebene der Datenantenne überlappt.

12. System nach einem der Ansprüche 8 bis 10, wobei die Datenantenne konzentrisch zur Leistungsantenne ist.

13. System nach einem der vorhergehenden Ansprüche, wobei der drahtlose Energieempfänger einen Nahfeldkommunikations-Energieempfänger umfasst und der drahtlose Datenempfänger einen Nahfeldkommunikations-Datenetikettenleser umfasst.

14. Verfahren, das Folgendes umfasst:
Erfassen eines Datenverfügbarkeitsereignisses auf einem Gerät, wobei:
das Datenverfügbarkeitsereignis eine Verfügbarkeit von Daten anzeigt, die für das Gerät drahtlos verfügbar sind,
das Gerät einen drahtlosen Energieempfänger umfasst, der mit einer Energieantenne gekoppelt ist,
die Vorrichtung einen drahtlosen Datenempfänger enthält, der mit einer Datenantenne gekoppelt ist, und
die Vorrichtung einen Schalter enthält, der den drahtlosen Datenempfänger selektiv mit der Datenantenne koppelt;
als Reaktion auf das Erfassen des Datenverfügbarkeitsereignisses den Schalter betätigt, um den drahtlosen Datenempfänger selektiv mit der Datenantenne zu koppeln; und
eine Verbindung zwischen der Vorrichtung und einer drahtlosen Datenquelle über die Datenantenne initiiert,
wobei das Verfahren **dadurch gekennzeichnet ist, dass**
das Datenverfügbarkeitsereignis die Erkennung des Einführens des Geräts in eine Tasche eines Kleidungsstücks neben einem Datenetikett umfasst,

15. Verfahren nach Anspruch 14, wobei das Datenverfügbarkeitsereignis ferner eine Erkennung des Kontakts mit der Haut eines Benutzers umfasst.

## Revendications

1. Système comprenant :
une antenne de puissance ;
un récepteur de puissance sans fil couplé à l'antenne de puissance ;
une antenne de données ;
un récepteur de données sans fil couplé à l'antenne de données ;
un commutateur couplant de façon sélective l'antenne de données au récepteur de données sans fil ; et
un circuit de commande configuré pour actionner le commutateur pour déconnecter l'antenne de données du récepteur de données sans fil en réponse à une puissance qui est reçue par le récepteur de puissance sans fil par l'intermédiaire de l'antenne de puissance, **caractérisé en ce que**
le circuit de commande est configuré en outre pour répondre à un événement de disponibilité de données, en connectant l'antenne de données au récepteur de données sans fil au moyen du commutateur, et en envoyant activement un ping pour une étiquette de données lisible avec le récepteur de données sans fil,
dans lequel l'événement de disponibilité de données comprend au moins un parmi (i) une détection de contact d'au moins une partie du système avec la peau d'un utilisateur, ou (ii) une détection d'une poche de vêtement autour d'au moins une partie du système.

2. Système selon la revendication 1, dans lequel le circuit de commande est configuré pour actionner le commutateur pour connecter l'antenne de données au récepteur de données sans fil quand une quantité de puissance reçue par le récepteur de puissance sans fil est inférieure à un seuil prédéterminé.

3. Système selon l'une des revendications précédentes, dans lequel le circuit de commande est configuré pour passer par défaut à un mode de réception de puissance avec le commutateur dans une position ouverte.

4. Système selon l'une des revendications précédentes, comprenant en outre un appareil de surveillance physiologique.

5. Système selon la revendication 4, dans lequel l'appareil de surveillance physiologique comprend une batterie chargée au moins en partie par le récepteur de puissance sans fil.

6. Système selon l'une des revendications 4 à 5, dans lequel l'appareil de surveillance physiologique comprend un contrôleur configuré pour actionner l'appareil de surveillance physiologique en réponse à des données reçues par le récepteur de données sans fil.

7. Système selon la revendication 6, dans lequel les données comprennent au moins un parmi un type de vêtement et un emplacement du corps de l'utilisateur.

8. Système selon l'une des revendications précédentes, dans lequel l'antenne de puissance est une antenne à bobine plane.

9. Système selon l'une des revendications précédentes, dans lequel l'antenne de données est une antenne à bobine plane.

10. Système selon l'une des revendications 8 à 9, dans lequel l'antenne de donnés est dans un plan parallèle à l'antenne de puissance.

11. Système selon l'une des revendications 8 à 9, dans lequel l'antenne de donnés chevauche l'antenne de puissance le long d'un axe perpendiculaire à un plan de l'antenne de données.

12. Système selon l'une des revendications 8 à 10, dans lequel l'antenne de donnés est concentrique avec l'antenne de puissance.

13. Système selon l'une des revendications précédentes, dans lequel le récepteur de puissance sans fil comprend un récepteur de puissance de communication en champ proche et le récepteur de données sans fil comprend un lecteur d'étiquette de données de communication en champ proche.

14. Procédé comprenant :
la détection d'un événement de disponibilité de données sur un dispositif, dans lequel :
l'événement de disponibilité de données indique une disponibilité de données disponibles sans fil pour le dispositif,
le dispositif comprend un récepteur de puissance sans fil couplé à une antenne de puissance, le dispositif comprend un récepteur de données sans fil couplé à une antenne de données, et le dispositif comprend un commutateur couplant de façon sélective le récepteur de données sans fil à l'antenne de données ;
en réponse à la détection de l'événement de disponibilité de données, l'actionnement du commutateur pour coupler de façon sélective le récepteur de données sans fil à l'antenne de données ; et
l'initiation d'une connexion entre le dispositif et une source de données sans fil par l'intermédiaire de l'antenne de données,
le procédé étant **caractérisé en ce que**
l'événement de disponibilité de données comprend une détection d'une insertion du dispositif dans une poche d'un vêtement adjacente à une étiquette de données.

15. Procédé selon la revendication 14, dans lequel l'événement de disponibilité de données comprend en outre une détection de contact avec la peau d'un utilisateur.
